(19) Europäisches Patentamt
European Patent Office
Office européen des brevets

(11) EP 3 354 258 A1

(12) # EUROPEAN PATENT APPLICATION

(43) Date of publication:
**01.08.2018 Bulletin 2018/31**

(21) Application number: **17305105.3**

(22) Date of filing: **31.01.2017**

(51) Int Cl.:
***A61K 9/00*** *(2006.01)*
***A61K 47/36*** *(2006.01)*
***A61K 38/10*** *(2006.01)*
***A61L 27/20*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **Teoxane SA**
**1203 Geneva (CH)**

(72) Inventors:
- **MEUNIER, Stéphane**
  **01710 THOIRY (FR)**
- **BOURDON, François**
  **74240 GAILLARD (FR)**

(74) Representative: **Nony**
**11 rue Saint-Georges**
**75009 Paris (FR)**

(54) **USE OF A COHESIVE GEL AS A MATRIX MATERIAL IN A PERIODONTAL POCKET**

(57) The present invention relates to a sterile and injectable gel comprising at least one crosslinked hyaluronic acid or a salt thereof and at least one non-crosslinked hyaluronic acid or a salt thereof for use in preventing and/or treating periodontal diseases and/or associated troubles and disorders, wherein said gel has a cross-over stress greater than or equal to 400 Pa and/or a compressive strength greater than or equal to 20 N.s.

EP 3 354 258 A1

**Description**

## FIELD OF THE INVENTION

The present invention relates to the use of a sterile and injectable gel comprising at least one crosslinked hyaluronic acid or a salt thereof and at least one non-crosslinked hyaluronic acid or a salt thereof, said gel having improved mechanical properties, especially in terms of cohesivity, as a matrix material in a periodontal pocket and thus, for treating periodontal diseases and associated disorders.

## DESCRIPTION OF RELATED ART

The present invention relates to the field of dental surgery in humans or animals, especially of the treatment of periodontal diseases and of associated disorders.

"Periodontal disease" is a generic name used to describe inflammatory disease of the periodontium, the tissue surrounding and securing teeth to the jawbone. The periodontium consists in the cementum, periodontal ligaments and the gingiva (gum), which includes the alveolar bone and the soft tissue covering it. Periodontal disease is the leading cause of tooth loss in the adult population (Anderson's Pathology, p.2000, John M. Kissane ed., 9th ed. (1992)).

"Gingivitis" is an accumulation of bacterial plaque in the gap between the gingiva and the tooth. As the disease progresses, a periodontal pocket is established below the gingival margin, thus prolonging and promoting the inflammatory process. Successive inflammatory reactions result in the progressive erosion of the tooth-supporting tissues, i.e., the collagenous fibres making up the periodontal ligament and the bone pocket in which the tooth sits (reviewed in Anderson's Pathology, pp. 1999-2000, John M. Kissane ed., 9th ed. (1992); Shafer et al, A Textbook of Oral Pathology, 4th ed. (1983)). As the disease progresses, the pockets deepen and more gum tissue and bone are destroyed. Eventually, teeth can become loose and may have to be removed.

In addition to dental disease associated with periodontal diseases and tooth decay, tooth developmental defects caused by severe malnutrition, genetic defects such as dentinogenesis imperfecta, trauma or drugs use can all lead to tooth loss.

Dental prostheses are used in patients who have partially or entirely lost their teeth.

Edentulous bone, namely alveolar bone without teeth embedded, progressively loses its volume, since no stress is transferred to the bone, as there is no tooth root or implant to transfer the forces of mastication to the bone. As a result of the diminishing volume, the contour and architecture of the alveolar ridge changes. The same happens with dentures, and the phenomenon of bone resorption is even more pronounced if dentures are used, and this is the reason why the dentures gradually lose their fit.

Biomaterials comprising hyaluronic acid, eventually in a crosslinked form, are already developed and commercialized in dental surgery, notably in the treatment of periodontal diseases.

In this regard, it may be notably mentioned the products disclosed in the documents WO 2009/144708 and WO 2014/056723.

In general, one can say that these biomaterials for dental surgery are used by mainly creating a filling/increasing of the concerned tissues.

Actually, with current available products containing hyaluronic acid, eventually in a crosslinked form, intended to treat periodontal diseases by their administering in the periodontal pockets, it is observed that due to poor structural and mechanical properties, a significant amount of the administered product (up to 98%) is actually removed in the hours following said administration.

Therefore, several administrations have to be considered on periods of time relatively reduced.

In this connection, providing to practitioners biocompatible formulations ready for use and easily manageable, having remarkable mechanical properties, particularly in terms of cohesivity, being suitable for injection for therapeutic purposes in the dental field, especially in the treatment of periodontal diseases, overcoming the above-mentioned disadvantages and which also do not involve any adverse effects remains a constant purpose.

Also, making available to practitioners more effective biocompatible formulations for such treatments remains a constant purpose.

The present invention is precisely intended to meet these expectations.

## DETAILED DESCRIPTION OF THE INVENTION

Thus, the invention relates to the use of a sterile and injectable gel comprising at least one crosslinked hyaluronic acid or a salt thereof and at least one non-crosslinked hyaluronic acid or a salt thereof, in a periodontal pocket wherein said gel has a cross-over stress greater than or equal to 400 Pa and/or a compressive strength greater than or equal to 20 N.s.

More particularly, the invention relates to a sterile and injectable gel comprising at least one crosslinked hyaluronic acid or a salt thereof and at least one non-crosslinked hyaluronic acid or a salt thereof for use in preventing and/or treating periodontal diseases and/or associated troubles and disorders, wherein said gel has a cross-over stress greater than or equal to 400 Pa and/or a compressive strength greater than or equal to 20 N.s, and preferably an extrusion force lower than or equal to 25 N, in particular via a 27G needle.

As below-detailed, the advantageous technical effects of a gel according to the present invention are attached to its specific intrinsic properties like its specific cohesivity and extrusion force.

The term "cohesivity" refers to the mechanical resistance of the gel and to its capacity to stay attached to itself i.e. its resistance to cutting and its ability to be elongated or compressed without to be separated into pieces.

Said cohesivity can be assessed by the parameters like the cross-over stress, measured by oscillatory rheology with stress sweep, and the compressive strength, measured by a compression resistance test in particular as detailed here-after.

The higher the cross-over stress is, the more the gel is able to keep its mechanical properties on a wide range of stress and deformation.

In particular, the higher the compressive strength is, the higher the gel can resist to the stress induced by a normal compression force.

Therefore, a gel according to the present invention is highly cohesive.

In particular, a gel according to the invention has a cross-over stress greater than or equal to 400 Pa or a compressive strength greater than or equal to 20 N.s.

Preferably, it has a cross-over stress greater than or equal to 400 Pa and a compressive strength greater than or equal to 20 N.s.

Thanks to these specific properties, a gel according to the invention exhibits an adequate residence time at the injection site and an adequate mechanical resistance for promoting its therapeutic and/or non-therapeutic effect.

Furthermore, the combination of crosslinked and non-crosslinked hyaluronic acids also allows to achieve gels having also a good injectability, i.e. a reduced extrusion force, without altering this improved cohesivity.

Thus, a gel according to the invention unexpectedly exhibits, simultaneously to its good cohesivity, a low extrusion force.

One skilled in the art understands that high extrusion forces can lead to lack of control during injection, difficulties or even impossibility to inject. Such lack of control may result in additional risks and/or pain for the patient.

Advantageously, a gel according to the present invention exhibits excellent extrusion force and is therefore easier to use for injection by a practitioner via a fine needle. Therefore, the administration of a gel according to the invention is easy and also significantly safer and less harmful for the patient. Naturally, the gel of the invention may be also directly injected inside the periodontal pockets. This specific protocol of administration allows avoiding any painful sensation for the patient associated with the introduction of a needle inside the gum,

In particular, a gel according to the invention is injectable via a fine hypodermic needle, in particular with a diameter of less than 18 G, or even less than 25 G.

Therefore, according to a preferred embodiment, a gel according to the invention has advantageously a cross-over stress greater than or equal to 400 Pa, a compressive strength greater than or equal to 20 N.s and an extrusion force lower than or equal to 25 N, in particular via a 27G needle, more specifically according to the conditions defined later in this document ("Materials and Methods").

The complying to both requirements, good cohesivity and low extrusion force, allows to achieve gels able to act as an efficient matrix material for periodontal pocket.

As already stated, the gel of the invention remains at the injection site, i.e. the treated periodontal pocket, on a significantly more important period of time than conventional gels. Thus, it not only fills the void or cavity of the periodontal pocket but further advantageously acts therein as a matrix material allowing to promote the construction/regeneration of the tissues originally present at the location of the treated periodontal pockets. As a consequence, a gel of the present invention allows improving the adhesion of the gum with the tooth surrounded by the so-treated periodontal pocket.

Accordingly, the invention also relates to a sterile and injectable gel comprising at least one crosslinked hyaluronic acid or a salt thereof and at least one non-crosslinked hyaluronic acid or a salt thereof for use in filling void in a periodontal pocket, wherein said gel has a cross-over stress greater than or equal to 400 Pa and/or a compressive strength greater than or equal to 20 N.s, and preferably an extrusion force lower than or equal to 25 N, in particular via a 27G needle.

The invention also relates to a sterile and injectable gel comprising at least one crosslinked hyaluronic acid or a salt thereof and at least one non-crosslinked hyaluronic acid or a salt thereof for use in reducing the mobility of at least one tooth to the periodontal pocket and/or improving the adhesion of the gum with at least one tooth, wherein said gel has a cross-over stress greater than or equal to 400 Pa and/or a compressive strength greater than or equal to 20 N.s, and preferably an extrusion force lower than or equal to 25 N, in particular via a 27G needle.

It also relates to a sterile and injectable gel comprising at least one crosslinked hyaluronic acid or a salt thereof and at least one non-crosslinked hyaluronic acid or a salt thereof for use as a matrix material in a periodontal pocket, wherein said gel has a cross-over stress greater than or equal to 400 Pa and/or a compressive strength greater than or equal to 20 N.s, and preferably an extrusion force lower than or equal to 25 N, in particular via a 27G needle.

In particular, a gel according to the invention may be also used as a matrix vehicle for administering and liberating, in the periodontal pocket, an active material useful for preventing and/or treating periodontal diseases and/or associated troubles and disorders. It may also constitute a matrix support for the regeneration of cells in the periodontal pocket.

The sterile and injectable gel according to the present invention may be used for therapeutic or non-therapeutic purpose.

Therefore, the invention also relates to the use of a sterile and injectable hydrogel comprising at least one crosslinked hyaluronic acid or a salt thereof and at least one non-crosslinked hyaluronic acid or a salt thereof for preventing and/or treating periodontal diseases and/or associated troubles and disorders, wherein said gel has a cross-over stress greater than or equal to 400 Pa and/or a compressive strength greater than or equal to 20 N.s, and preferably an extrusion force lower than or equal to 25 N, in particular via a 27G needle.

It also relates to the use of a sterile and injectable gel comprising at least one crosslinked hyaluronic acid or a salt thereof and at least one non-crosslinked hyaluronic acid or a salt thereof for filling, in particular as a matrix material, a periodontal pocket, wherein said gel has a cross-over stress greater than or equal to 400 Pa and/or a compressive strength greater than or equal to 20 N.s, and preferably an extrusion force lower than or equal to 25 N, in particular via a 27G needle.

It also relates to the use of a sterile and injectable gel comprising at least one crosslinked hyaluronic acid or a salt thereof and at least one non-crosslinked hyaluronic acid or a salt thereof for reducing the mobility of at least one tooth to the periodontal pocket and/or improving the adhesion of the gum with at least one tooth wherein said gel has a cross-over stress greater than or equal to 400 Pa and/or a compressive strength greater than or equal to 20 N.s, and preferably an extrusion force lower than or equal to 25 N, in particular via a 27G needle.

The invention also relates to the use of a sterile and injectable gel comprising at least one crosslinked hyaluronic acid or a salt thereof and at least one non-crosslinked hyaluronic acid or a salt thereof for forming a medium appropriate to the regeneration of cells in a periodontal pocket wherein said gel has a cross-over stress greater than or equal to 400 Pa, and/or a compressive strength greater than or equal to 20 N.s, and preferably an extrusion force lower than or equal to 25 N, in particular via a 27G needle.

According to a specific embodiment, the gel of the instant invention is obtained by a method comprising at least the steps of:

a) contacting at least one crosslinked hyaluronic acid or a salt thereof with at least one non-crosslinked hyaluronic acid or a salt thereof, said crosslinked and non-crosslinked hyaluronic acid being, independently one from the other in a dry state or in a hydrated state, and

b) mixing them until homogenization, and where applicable adding an aqueous solution, for forming a hydrogel having a cross-over stress greater than or equal to 400 Pa and/or a compressive strength greater than or equal to 20 N.s, and preferably an extrusion force lower than or equal to 25 N, in particular via a 27G needle.

According to a first embodiment, the non-crosslinked hyaluronic acid and the crosslinked hyaluronic acid contacted in step a) are both in a hydrated state, in particular they are under the form of homogenous hydrogels that have preferably the same concentration in hyaluronic acid.

In particular, the homogenous hydrogel of crosslinked hyaluronic acid or a salt thereof has a cross-over stress greater than or equal to 400 Pa and/or a compressive strength greater than or equal to 20 N.s and an extrusion force greater than 25 N, in particular via a 27G needle.

According to a second embodiment, in step a) the non-crosslinked hyaluronic acid is provided diluted in an aqueous solution and the crosslinked hyaluronic acid is provided as a solid and dense three-dimensional network as obtained just after the crosslinking of the non-crosslinked hyaluronic acid raw material and before swelling to the desired concentration of hyaluronic acid.

According to a preferred embodiment, the hyaluronic acid content of a gel according to the present invention consists in one crosslinked hyaluronic acid or a salt thereof and one non-crosslinked hyaluronic acid or a salt thereof.

In particular, a gel according to the invention comprises from 2% to 3% by weight of hyaluronic acid, relative to the total weight of the gel, consisting in:

- from 90% to 97% by weight of crosslinked hyaluronic acid or a salt thereof having a degree of crosslinking ranging from 9% to 11%, relative to the total weight of hyaluronic acid, and
- from 3% to 10% by weight of non-crosslinked hyaluronic acid or a salt thereof, relative to the total weight of hyaluronic

acid.

According to another aspect, the invention relates to a process for improving the injectability of a gel comprising at least one crosslinked hyaluronic acid or a salt thereof and having a cross-over stress greater than or equal to 400 Pa and/or a compressive strength greater than or equal to 20 N.s and which value of cross-over stress and/or compressive strength have to be maintained greater than or equal respectively to 400 Pa and 20 N.s., comprising the step of adding to said gel an efficient amount of at least one gel of hyaluronic acid whose hyaluronic acid component consists in non-crosslinked hyaluronic acid or a salt thereof.
In particular, the gel of non-crosslinked hyaluronic acid or a salt thereof is added for reducing the extrusion force of the gel of cross-linked hyaluronic acid to a value lower than or equal to 25 N with a needle as thin as a 27G needle, in the conditions defined later in this document ("Materials and Methods").
For the purpose of the present invention,

- the term "efficient amount" relates to the amount of non-crosslinked hyaluronic acid or a salt thereof that is needed to improve the injectability, i.e. to reduce the extrusion force, of the gel while maintaining the cross-over stress to a value greater than or equal to 400 Pa.
- the term "non-crosslinked hyaluronic acid" or a salt thereof relates to the hyaluronic that was not submitted to a crosslinking reaction and which is added to the composition to reduce the extrusion force of the gel or used for preparing crosslinked hyaluronic acid gel.
- the term "sterile" relates to an environment ensuring the safety required for administration in or through the human tissues, in particular periodontal pockets. In particular, it is essential for the gel containing said compounds and which has to be administered in periodontal pockets, to be devoid of any viable microorganisms or contaminating body capable of initiating an adverse side reaction in the host organism.
- the term "injectable" is intended to denote a composition of which the mechanical properties are appropriate for the use considered, namely its administration in or through human tissues, in particular in periodontal pockets. Preferably, the term « injectable » refers to a gel with an extrusion force, through an appropriate, fine, needle, particularly a 27G needle, being less than or equal to 25 N, when measured as described hereinafter.
- the term "gel of hyaluronic acid" also called in the instant specification "hydrogel" relates to a homogenous aqueous and viscous solution of crosslinked and/or non-crosslinked hyaluronic acid.
- the term "homogeneous" means a physical state wherein the visual aspect is uniform and the physical properties, like for example viscosity or cross-over stress, are similar throughout the related gel or solution.
- the term "dry state" means a physical state wherein the crosslinked and/or non-crosslinked hyaluronic acid is a solid, free of aqueous medium. Examples of dry states are powders or fibres.
- the term "hydrated state" means a physical state wherein the crosslinked and/or non-crosslinked hyaluronic acid is diluted or swelled in an aqueous medium. Examples of hydrated states are gels or viscous solutions.

## Intrinsic properties of the gels according to the invention

As already specified here-above, the gel according to the present invention is particularly interesting with respect to specific intrinsic properties like more particularly its cohesivity.
The term "cohesivity" refers to the mechanical resistance of the gel, to its capacity to stay attached to itself, i.e. meaning the resistance to cutting and its ability to elongate or to compress without to be separated into pieces.
As already stated, the cross-over stress, measured by oscillatory rheology with stress sweep, and the compressive strength, measured by a compression resistance test may be used for assessing this cohesivity.
The higher the compressive strength is, the higher the gel can resist to the stress induced by a normal compression force.
Advantageously, a gel according to the present invention is able to keep its mechanical properties on a wider range of stress and deformation.

### Cross-over stress

A gel according to the invention has a cross-over stress greater than or equal to 400 Pa.
The cross-over stress relates to the stress at which the value of G', the elastic modulus, decreases and equals the value of G", the viscous modulus (that is to say the cross between G' and G" values). In other words, the cross-over stress is the stress (in Pa) when G'=G". The stress and deformation strain at this point are those starting from which a material, predominantly elastic at the lower stresses and deformation strains, enters the flow region. This translates the destructuration of the gel. It may be measured using a rheometer with a cone-plate geometry. Alternatively, the measurements may also be carried out using a two-parallel rough plates geometry, which enables a better reliability and

precision of cross-over measurements.

A convenient method for evaluating this cross-over stress is disclosed in the following section "Materials and Methods".

Compressive strength

Preferably, a gel according to the invention has a compressive strength greater than or equal to 20 N.s.

The compressive strength measures the mean resistance of a gel when it is compressed between two plates. The compressive strength is the time integration of the resistance force exerted by the gel in normal direction when placed between a moveable plate and a stationary plate; it is thus expressed in N.s.

The combination of crosslinked and non-crosslinked hyaluronic acids or salts thereof required in the gel according to the invention is relevant to provide a good injectability, ie. reduced extrusion force, while keeping a cross-over stress greater than or equal to 400 Pa and a compressive strength greater than or equal to 20 N.s.

A convenient method for evaluating this compressive strength is disclosed in the following section "Materials and Methods".

Extrusion force

Preferably, a gel according to the invention has an extrusion force lower than or equal to 25 N, in particular with a needle as thin as a 27G needle, in the conditions defined later in this document ("Materials and Methods").

The extrusion force allows to characterize the injectability of a product.

Lower the extrusion force is, better is its injectability.

The extrusion force of the gels is measured by extruding the gel from a syringe using a compression stand and a calibrated dynamometer.

A convenient method for evaluating this extrusion force is disclosed in the following section "Material and Method".

Resistance to the degradation (by incubation with $H_2O_2$)

In addition, a gel according to the invention has preferably a degradation rate to $H_2O_2$ lower than or equal to -30 %.

The resistance to the degradation of a gel may be assessed in considering the loss of elastic modulus when the gel is subjected to degradation by incubation with $H_2O_2$. This loss characterizes a degradation to oxidative stress and may be used as an indicator of the lasting of the gel in the body.

**Hyaluronic acid**

As already specified, a gel according to the invention comprises at least one crosslinked hyaluronic acid or a salt thereof and at least one non-crosslinked hyaluronic acid or a salt thereof.

According to a preferred embodiment, the total hyaluronic acid content of the gel consists in one crosslinked hyaluronic acid or a salt thereof and one non-crosslinked hyaluronic acid or a salt thereof.

In particular, a sterile and injectable gel according to the invention may comprise from 1% to 4% by weight, preferably from 2% to 3% by weight of hyaluronic acid, relative to the total weight of said gel.

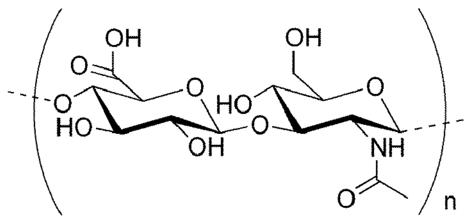

As above-mentioned, hyaluronic acid (also called hyaluronan or hyaluronate) is a linear non-sulphated glycosaminoglycan composed of repeating units of D-glucuronic acid and N-acetyl-D-glucosamine (Tammi R., Agren UM., Tuhkanen AL., Tammi M. Hyaluronan metabolism in skin. Progress in Histochemistry & Cytochemistry 29 (2): 1. -81, 1994).

As above-mentioned, the present invention considers the implementation of hyaluronic acid as such but also as a salt thereof.

Therefore, the hyaluronic acid in accordance with the invention may be more particularly chosen from physiologically acceptable salts such as the sodium salt, the potassium salt, the zinc salt, the silver salt and mixtures thereof, preferably the sodium salt.

One particularly preferred salt of hyaluronic acid is sodium hyaluronate (NaHA).

All number herein expressing "molecular weight" of hyaluronic acid are to be understood as indicating the weight average molecular weight (Mw) in Daltons (Da).

The molecular weight of hyaluronic acid is calculated from an intrinsic viscosity measurement using the following Mark-Houwink relation:

$$\text{Intrinsic Viscosity } (m^3/kg) = 9.78 \times 10^{-5} \times Mw^{\,0.690}.$$

Crosslinked hyaluronic acid

A crosslinked hyaluronic acid can be obtained by any conventional technique of crosslinking with at least one crosslinking agent. In particular, it may be referred to the US Pat. No. 9 353 194. Preferably, the crosslinked hyaluronic acid or salt thereof is obtained by crosslinking at least one non-crosslinked hyaluronic acid or a salt thereof with at least one chemical crosslinking agent.

The term "crosslinking agent" relates to any compound capable of inducing a crosslinking between the chains of the hyaluronic acid.

The choice of this crosslinking agent clearly falls within the competence of a person skilled in the art.

A crosslinking agent in accordance with the invention is preferably a difunctional (or bifunctional) or a multifunctional crosslinking agent.

More particularly, an epoxy crosslinking agent according to the present invention may be preferably selected from the group consisting of 1,4-butanediol diglycidyl ether (BDDE), diepoxyoctane or 1,2-bis(2,3-epoxypropyl)-2,3-ethylene, 1,4-bis(2,3-epoxypropoxy)butane, 1,4-bisglycidyloxybutane, 1,2-bis(2,3-epoxypropoxy)ethylene, and 1-(2,3-epoxypropyl)-2,3-epoxycyclohexane and mixtures thereof.

Non-epoxy crosslinking agent, like for example endogenous polyamines may be also used.

Preferably, a crosslinking agent according to the invention is 1,4-butanediol diglycidyl ether (BDDE).

The crosslinked hyaluronic acid as recovered just after this crosslinking is as a solid and dense three-dimensional network of hyaluronic acid. It is subsequently submitted to a swelling and neutralizing step for forming the expected homogenous gel of crosslinked hyaluronic acid.

Regarding the crosslinked hyaluronic acid or a salt thereof, it has preferably a degree of crosslinking ranging from 0.1 to 20%, preferably from 3 to 12%, more preferably from 9 to 11%.

The term "degree of crosslinking" is defined by the percentage of the weight ratio between the crosslinking agent to the hyaluronic acid used for the crosslinking, i.e. $m_{(BDDE)}/m_{(HA\ to\ be\ crosslinked)}$ multiplied by 100.

Preferably, the non-crosslinked hyaluronic acid or a salt thereof used for preparing the crosslinked hyaluronic acid or a salt thereof has a high average molecular weight, preferably ranging from 0.3 MDa to 10 MDa, preferably from 0.5 MDa to 4 MDa., more preferably from 0.5 MDa to 2 MDa.

An effective amount of crosslinked hyaluronic acid or a salt of thereof used for preparing a gel according to the invention may range from 80% to 99.9% by weight, preferably from 90% to 99% by weight, more preferably from 95% to 97% by weight relative to the total weight of hyaluronic acid in the gel.

Non-crosslinked hyaluronic acid

For the purpose of the present invention, the term "non-crosslinked" or "uncrosslinked" is understood to mean a hyaluronic acid which is not involved in a crosslinking step process. Non-crosslinked hyaluronic acid generally remains water soluble.

The non-crosslinked hyaluronic acid according to the invention has preferably an average molecular weight ranging from 0.3 MDa to 10 MDa, preferably from 0.5 MDa to 4 MDa, more preferably from 0.5 to 2 MDa.

An efficient amount of non-crosslinked hyaluronic acid or a salt of thereof used for preparing a gel according to the invention may range from 0.1% to 20% by weight, preferably from 1% to 10% by weight, more preferably from 3% to 5% by weight relative to the total weight of hyaluronic acid in the gel.

It has to be noticed that the efficient amount of non-crosslinked hyaluronic acid depends on the hyaluronic acid concentration and on the degree of crosslinking of the gel.

According to a preferred embodiment, the weight ratio of non-crosslinked hyaluronic acid over crosslinked hyaluronic acid is ranging from 0.001 to 0.25, preferably from 0.01 to 0.11, more preferably from 0.03 to 0.05.

According to a particular embodiment, a gel according to the invention comprises from 1% to 4% by weight of hyaluronic acid, relative to the total weight of said gel, wherein the hyaluronic acid consists in:

- from 80% to 99.9% by weight of at least one crosslinked hyaluronic acid or a salt thereof having a degree of crosslinking ranging from 0.1 to 20%; relative to the total weight of hyaluronic acid, and
- from 0.1% to 20% by weight, of at least one non-crosslinked hyaluronic acid or a salt thereof, relative to the total weight of hyaluronic acid.

According to another embodiment, a gel according to the invention comprises from 2% to 3% by weight of hyaluronic acid relative to the total weight of said gel, wherein the hyaluronic acid consists in:

- from 90% to 99% by weight of at least one crosslinked hyaluronic acid or a salt thereof having a degree of crosslinking ranging from 3% to 12%, relative to the total weight of hyaluronic acid, and
- from 1% to 10% by weight of at least one non-crosslinked hyaluronic acid or a salt thereof, relative to the total weight of hyaluronic acid.

According to another embodiment, a gel according to the invention comprises from 2% to 3% by weight of hyaluronic acid relative to the total weight of said gel, wherein the total hyaluronic acid content consists in:

- from 95% to 97% by weight of one crosslinked hyaluronic acid or a salt thereof and having a degree of crosslinking ranging from 9% to 11%, relative to the total weight of hyaluronic acid, and
- from 3% to 5% by weight of one non-crosslinked hyaluronic acid or a salt thereof, relative to the total weight of hyaluronic acid.

## Additional compounds

According to particular embodiments, a gel according to the invention may further comprise additional compound(s).
For obvious reasons, these additional compounds, and also their respective amounts, are adapted to not be deleterious with respect to the properties of a gel according to the invention.
Advantageously, in view of the considered technical field, a gel according to the invention may further comprise one or more additional compound(s) having a positive impact with respect to the prevention and/or treatment of periodontal diseases and associated disorders. It may be in particular used as a vehicle for administering inside the periodontal cavity, an active material and in particular a therapeutic agent like an anesthetic agent, an antibiotic agent, an antioxidant, a vitamin and a mixture thereof.
Other examples of additional compounds may be also mineral charges, such as hydroxyapatite or a ceramic material.

## Method for preparing a sterile and injectable gel according to the invention

The gel of the instant invention may be obtained by a method comprising at least the steps of

a) contacting, at least one crosslinked hyaluronic acid or a salt thereof with at least one non-crosslinked hyaluronic acid or a salt thereof, said crosslinked and non-crosslinked hyaluronic acid being, independently one from the other, in a dry state or in a hydrated state, preferably in a hydrated state, and
b) mixing them until homogenization, and where applicable adding an aqueous solution, for forming a hydrogel keeping a cross-over stress greater than or equal to 400 Pa and/or a compressive strength greater than or equal to 20 N.s, and preferably an extrusion force lower than or equal to 25 N, in particular via a 27G needle.

According to a first embodiment, the non-crosslinked acid and the crosslinked hyaluronic acid contacted in step a) are both under the form of homogenous hydrogels, and preferably of same concentration of hyaluronic acid.
According to a second embodiment, in step a) the non-crosslinked acid is provided diluted in an aqueous solution and the crosslinked hyaluronic acid is provided as a solid and dense three-dimensional network as obtained just after the crosslinking of a non-crosslinked hyaluronic acid raw material and before swelling to the desired concentration of hyaluronic acid.
According to this embodiment, the step of swelling the crosslinked hyaluronic acid is performed at the same time than the step b) of mixing both crosslinked and non-crosslinked hyaluronic acids. This step b) is performed until a homogenous gel according to the invention is formed.
The homogenization step b) may be performed by one or several conventional mean(s) that may be chosen by one skilled in the art. In particular, the homogenization may take place in an airtight and deformable pocket, and/or with a three-dimensional mixer.

The adjustment of the appropriate homogenization time for obtaining an aqueous hyaluronic acid gel which is sufficiently homogeneous without degrading the hyaluronic acid is part of the general knowledge of those skilled in the art. It particularly depends on the nature of the hyaluronic acid, and more particularly on its molecular weight, on its concentration, on the operating conditions within the aqueous medium and also on the homogenization device used.
The homogenization is considered to be satisfactory when the solution obtained has a homogeneous colouration, without agglomerates, and a uniform viscosity. It can advantageously be carried out under mild operating conditions so as to prevent degradation of the hyaluronic acid chains.
Indeed, this step is all the more important when the hyaluronic acid has a high molecular weight.
The gel obtained at the end of the process of the invention as previously described may not be directly injectable because of the possible presence of crosslinking agent residues, or else because of its physiological and/or pH conditions which are incompatible with use in the fields of application considered above.
Consequently, several additional steps, known to those skilled in the art, are generally carried out in order to obtain an injectable hydrogel.
More particularly, a step of neutralizing and swelling this gel to the desired hyaluronic acid concentration may be needed in order to give it its implant qualities. The chains of the polysaccharide network are then stretched and hydrated, while the pH is brought to that of the dermis.
For even higher purity, a purification step can be carried out in order to bring the gel to a physiologically acceptable salt concentration and/or to remove the crosslinking agent not attached to the polysaccharide and also any reaction intermediate or auxiliary agent.
Advantageously, this purification step can be carried out by means of one or more dialysis baths.
Finally, the resulting gel, which may be called hydrogel, may then be packaged in syringes. The syringes may then be sterilized. For example, the compositions are sterilized by autoclaving at an effective temperature and pressure, determined in order to preserve the rheological properties of the gel. This determination falls within the competence of the person skilled in the art. The sterilized syringes are then packaged along with a fine gauge needle and/or a specific blunt needle for use by a physician.
Due to the finality of the gels according to the invention, said syringes may comprise appropriate dental needles. The determination of the appropriate dental needles depends on the administration mode chosen and falls within the competence of the person skilled in the art. Said needle may be straight or curved. When the product is administered directly inside the periodontal pocket, said needle is preferably chosen among blunt needles such as those commercialized under the name *"ANEL special hypodermic blunt needles for dental purpose*" by ANEL.

**Administration of the gel**

A gel according to the invention can be injected in periodontal pockets using any of the known methods in the art.
Particularly, a gel according to the invention may be administered by means of any injection device appropriate in the case of injection of material in the periodontal pockets.
In known manner, the syringe can be operated manually by the practitioner or by a syringe holder as a gun.
For obvious reasons, a gel according to the present invention is administered by injection in a periodontal pocket, in an effective amount.
The term "effective amount" relates to an appropriate amount to obtain the desired technical effect, namely mainly to fill the treated periodontal pocket. For obvious reasons, this effective amount depends on the depth and on the width of the treated periodontal pocket.
Adjusting the amount of gel according to the present invention falls within the competence of a person skilled in the art.

**Applications of the gel**

As illustrated in the examples, a gel according to the invention i.e. having required intrinsic properties in terms of cross-over stress, compressive strength, extrusion force and also resistance to the degradation, has very interesting results with respect to the reduction of the tooth mobility.
It also allows improving the adhesion of the gum with the tooth surrounded by the treated periodontal pocket and it is particularly interesting for preventing and/or treating periodontal diseases and/or associated disorders.
More widely, a gel according to the invention is intended to:

- prevent and/or treat periodontal diseases, gingivitis,
- improve state of moderate to severe mucositis, gingivitis and periodontitis,
- come in complement of other conventional periodontal treatment,
- fill depressives papillaes,

- improve intra oral tissue regeneration,
- preserve socket,
- guide tissue regeneration,
- regenerate the oral mucosa and the periodontium, and/or
- support accompaniment of systematic periodontal therapy.

Thus, the present invention also relates to a method for treating periodontal diseases and/or associated troubles and disorders, comprising a step of injecting in a periodontal pocket of a patient in need thereof a sterile and injectable gel comprising at least one crosslinked hyaluronic acid or a salt thereof and at least one non-crosslinked hyaluronic acid or a salt thereof, said gel having a cross-over stress greater than or equal to 400 Pa and/or a compressive strength greater than or equal to 20 N.s, and preferably an extrusion force lower than or equal to 25 N, in particular via a 27G needle.
The present invention also relates to a method for reducing the mobility of a tooth, comprising a step of injecting in a periodontal pocket a sterile and injectable gel comprising at least one crosslinked hyaluronic acid or a salt thereof and at least one non-crosslinked hyaluronic acid or a salt thereof, said gel having a cross-over stress greater than or equal to 400 Pa and/or a compressive strength greater than or equal to 20 N.s, and preferably an extrusion force lower than or equal to 25 N, in particular via a 27G needle.
The present invention also relates to a method for improving the adhesion of the gum with the tooth, comprising a step of injecting in a periodontal pocket a sterile and injectable gel comprising at least one crosslinked hyaluronic acid or a salt thereof and at least one non-crosslinked hyaluronic acid or a salt thereof, said gel having a cross-over stress greater than or equal to 400 Pa and/or a compressive strength greater than or equal to 20 N.s, and preferably an extrusion force lower than or equal to 25 N, in particular via a 27G needle.
Advantageously, positive effects regarding the prevention and/or the treatment of periodontal diseases and/or related disorders can be observed from the first administration of a gel according to the invention in periodontal pocket(s).
According to a specific embodiment, a drug may be also present in a gel according to the invention. As stated previously, it may be a drug suitable for the treatment of periodontal diseases and/or associated troubles and disorders.
Throughout the description, including the claims, the expression "comprising a" should be understood as being synonymous with "comprising at least one", unless specifically stated otherwise.
The expressions "between ... and ..." and "ranging from ... to ..." should be understood to mean that the limits are inclusive, unless specified otherwise.
The term about in the context of numerical values will be readily understood by a person skilled in the art, and preferably means that specific values may be modified by ± 10%. As regard endpoints of ranges, the modifier "about" preferably means that the lower endpoint may be reduced by 10% and the upper endpoint increased by 10%. It is also contemplated that each numerical value or range disclosed in this application can be absolute, ie that the modifier "about" can be deleted.
Unless otherwise indicated, all numbers expressing quantities of ingredients, properties such as molecular weight, reaction conditions, and so forth, used in the specification and claims are to be understood as being modified in all instances by the term "about". Accordingly, unless indicated to the contrary, the numerical parameters set forth in the specification and attached claims are approximations that may vary depending upon the desired properties sought to be obtained by the present invention. At the very least, and not as an attempt to limit the application of the doctrine of equivalents to the scope of the claims, each numerical parameter should at least be construed in the light of the number of reported significant digits and by applying ordinary rounding techniques.
The following examples are given by way of non-limiting illustration of the invention.

## MATERIALS AND METHODS

### Measurements protocols

For the purpose of the following measurements, the ambient temperature corresponds to a temperature of 22°C±3°C.
The cross-over measurements are carried out at ambient temperature, at a frequency of 1 Hz, with a stress sweep, using a Thermo Haake RS6000 rheometer with a cone-plate geometry of 1°/35 mm diameter. G' and G" are recorded at an applied deformation stress of 5 Pa, i.e. in the viscoelasticity range where G' and G" remain stable. The value of the cross-over stress, ie. when G' decreases to equal the viscous modulus G", is then also recorded.
The compressive strength is measured at ambient temperature, with 2.5 g of the considered gel disposed between two circular plates of 35 mm in diameter, with an initial "air gap" of 10 mm. A compression of 70% of the height of the air gap is applied to the speed of 0.1 mm/s. The compressive strength is the time integration of the resistance force of the gel.
The extrusion force of the gels is measured by extruding at ambient temperature, the gel from a 1mL long

format syringe, with an internal diameter of 6.5 mm and with an ultra-thin-walled needle (TSK 27G) using a compression stand (speed of descent: 12.5 mm/min) and a calibrated dynamometer.

The resistance to the degradation of a gel is assessed in considering the loss of elastic modulus when the gel is subjected to degradation by incubation with $H_2O_2$. This loss characterizes a degradation to oxidative stress.

In this regard, in 2 g of the considered gel, 67 μL of a solution of $H_2O_2$ at 10% w/w is added and the mixture is homogenized by 10seconds stirring with a mixer of vortex type, an incubation for 24 hours at 37 °C is carried out. The G' of the considered gel is measured before degradation and after degradation at ambient temperature, at a frequency of 1 Hz, with a stress sweep, using a Thermo Haake RS6000 rheometer with a cone-plate geometry of 1°/35 mm diameter.

## EXAMPLE 1:

### Preparation of a gel according to the invention

A gel according to the invention was prepared by mixing a gel of a crosslinked hyaluronic acid with a gel of a non-crosslinked hyaluronic acid.

The non-crosslinked hyaluronic acid has an average molecular weight of about 1.5 MDa and the crosslinked hyaluronic acid is also obtained from a non-crosslinked hyaluronic acid having an average molecular weight of about 1.5 MDa.

Protocol for preparing a gel of the crosslinked hyaluronic acid

1. Preparation of a mixture of non-crosslinked hyaluronic acid in a 1% w/w solution of sodium hydroxide (see Table 1 below);
2. Addition of a crosslinking agent (BDDE) to said mixture (see Table 1 below) and homogenization of said mixture at ambient temperature for about 1h30, until a homogeneous viscous solution is obtained;
3. Incubation of the mixture obtained, at a temperature of from 45°C to 55°C, for 3 hours (± 15 min), in order to crosslink the chains of the hyaluronic acid; and
4. Neutralization and swelling of the mixture obtained by addition of an acidified phosphate buffer solution, and homogenization, so as to obtain a hydrogel containing crosslinked hyaluronic acid, with a pH between 6.8 to 7.8.

The characteristics of the crosslinked hyaluronic acid in gel A are summarized in Table 1.

Table 1

| | **HA concentration (dry matter, mg/ml)** | **Degree of crosslinking $m_{(BDDE)}/m_{(HA\ to\ be\ crosslinked)}$** |
|---|---|---|
| **Gel of crosslinked hyaluronic acid** | 25 | 10.5 |

Protocol for preparing the solution of the non-crosslinked hyaluronic acid

The non-crosslinked hyaluronic acid is added to a saline phosphate buffer solution at a concentration of 25 mg/ml and homogenized therein at ambient temperature for about 20 hours until a homogeneous viscous solution is obtained.

Protocol for preparing a Gel **A** according to the invention

The previous non-crosslinked hyaluronic acid solution is added to the previous gel of crosslinked hyaluronic acid in the ratio uncrosslinked HA / crosslinked HA (expressed in dry matter) given in Table 2 below. Their mixture is performed in an airtight and deformable pocket wherein it is homogenized at ambient temperature, until formation of a homogeneous gel.

Table 2

| | m(uncrosslinked HA solution)/m(crosslinked HA gel) |
|---|---|
| **Gel A** | 4/96 |

The so-formed homogenized gel is then packaged in 1 mL long format syringes having an internal diameter of 6.5 mm. The syringes are then sterilized by autoclaving at effective temperature and pressure, determined in order to preserve the rheological properties of the gel.

Characterization of the Gel A:

The compressive strength, the cross-over stress and the *in vitro* degradation to $H_2O_2$ of gel A were assessed according to the respective protocols disclosed above.
The results are presented in Table 3 below.

Table 3

| | Compressive strength (N.s) | Cross-over stress (Pa) | Degradation to $H_2O_2$ (%)* |
|---|---|---|---|
| **Gel A** | 25 | 537 | -20 |

Gel A has a cross-over stress over 400 Pa and thus complies with the requirements of the invention.
In view of the above, it is also noticed that the Gel A according to the invention advantageously displays a high resistance to the degradation to $H_2O_2$ since its loss of G' is lower than 30% of the initial value of G'.

**EXAMPLE 2:**

**Preparation of different gels**

Three gels according to the invention were prepared according to the protocol of preparation disclosed for the Gel A in Example 1. The proportions of the non-crosslinked HA solution over the crosslinked HA gel, the average molecular weight and the degree of crosslinking of the crosslinked hyaluronic acid are submitted in Table 4 below.
Three comparative gels were also prepared without including a non-crosslinked hyaluronic acid.
The extrusion force (in N), the cross-over stress (in Pa) and the compressive strength (in N.s) of these gels were assessed according to the respective protocols disclosed hereabove.
The results are presented in Table 4 below.

Table 4

| | Gel A-1 | Gel A-2 | Gel A-3 | Gel A-4 (Comparative) | Gel A-5 (Comparative) | Gel A-6 (Comparative) |
|---|---|---|---|---|---|---|
| **Non-crosslinked HA/Total HA content (%)** | 5 | 3 | 5 | 0 | 0 | 0 |
| **Degree of crosslinking (%)** | 9.5 | 9.5 | 10.5 | 10.5 | 9.5 | 7.5 |
| **Mw HA used for preparing the crosslinked HA gel (MDa)** | 1.5 | 1.5 | 1.5 | 1.5 | 0.7 | 1.5 |
| **Mw HA used for preparing the non-crosslinked HA solution (MDa)** | 1.5 | 1.5 | 1.5 | N/A | N/A | N/A |
| **Extrusion force (N)** | 18 | 17 | 18 | 37 | 30 | 26 |
| **Cross-over stress (Pa)** | 451 | 514 | 478 | 700 | 488 | 526 |
| **Compressive strength (N.s)** | 20 | 21 | 23 | 29 | 16 | 13 |

Gels A-1, A-2 and A-3 exhibit a cross-over stress over 400 Pa, a compressive strength greater than or equal to 20 N.s, and an extrusion force lower than 25 N. A practitioner will therefore have no difficulty to inject said gels via a

fine needle. Said gels also probably have an adequate residence time at the injection site and an adequate mechanical resistance in a periodontal pocket of a patient.

Regarding the comparative gels, it may be noticed that:

Gel A-4, based on a crosslinked hyaluronic acid of same molecular weight and same crosslinking degree as the one of Gel A-3 exhibits a very high cross over and an acceptable compressive strength, however its extrusion force is far too high. This gel is therefore not suitable for injection by a practitioner via a fine needle.

Gels A-5 and A-6 both exhibit a cross-over stress over 400 Pa but their compressive strength is too low and their extrusion force is over 25 N, which is therefore non-suitable for injection by a practitioner via a fine needle.

Accordingly, even by modulating the degree of crosslinking of the crosslinked hyaluronic acid and the average molecular weight of the starting hyaluronic acid in order to achieve equivalent cross-over stress values, their compressive strength is unacceptable and the extrusion force remains too high for those gels.

## EXAMPLE 3:

### Clinical study

The objective of this study was to evaluate the efficacy of the Gel A of example 1, in periodontitis and gingivitis, through the improvement of tooth mobility.

This was uncontrolled, open-label, multi center, prospective case series to study the gel A in the treatment of gingivitis and periodontitis in healthy male and female subjects. Eligible subjects were treated on one or several teeth.

The gel has been administered directly in the periodontal pocket in a quantity necessary to fill said periodontal pocket, based on the investigator's judgment.

To be eligible for participation in the study the subjects had to fulfill the following criteria:

- Men or women, 18 years or older,
- Patients of grade 1 to 3 using Muhellmann's mobility index,
- Patients with a gum inflammatory index of grade 1 to 3,
- Patients with factors promoting periodontal problems: smoking, diabetes, etc.

In total, 12 subjects were included and treated in this study:

60.9% of the study participants were female and 39.1% were male. The age of the study subjects ranged from 24 to 80 years. No patient was diabetic, no patient was under a medical treatment, and no patient has an allergy. Most subjects did not smoke (1 subject was occasional smoker and 2 were dependent smokers).

The number of treated teeth by patient ranged from 1 tooth to 12 teeth.

| | **Gel A** N*=12 |
|---|---|
| N | 12 |
| missing data | 0 |
| mean (standard deviation) | 2.3 (1.22) |
| median | 2.5 |
| min , max | 1 , 4 |
| sum | 27 |
| *N = number of subjects | |

At initial treatment, the mean volume injected per tooth was 0.113 mL (± 0.1171 mL, SD).

| | Gel A n*=27 |
|---|---|
| n | 23 |
| missing data | 4 |
| mean (SD) | 0.113 (0.1171) |
| median | 0.100 |
| min, max | 0.03 , 0.60 |
| *n= number of teeth | |

Results

The grade of tooth mobility, on the Muhellmann's mobility index, before initial treatment and one month after injection, from the medical opinion, is given in the table below.

| | Gel A n*=27 | |
|---|---|---|
| **Inclusion (before treatment)** | | |
| Ankylosis | 3 | (11.1%) |
| Physiological mobility perceptible between two fingers | 12 | (44.4%) |
| Transverse mobility visible to the naked eye | 10 | (37.0%) |
| Transverse mobility > 1 mm | 2 | (7.4%) |
| Axial mobility | 0 | (0.0%) |
| **1 month after injection** | | |
| Ankylosis | 7 | (30.4%) |
| Physiological mobility perceptible between two fingers | 15 | (65.2%) |
| Transverse mobility visible to the naked eye | 1 | (4.3%) |
| Transverse mobility > 1 mm | 0 | (0.0%) |
| Axial mobility | 0 | (0.0%) |
| Missing data | 4 | |
| *n= number of teeth | | |

When considering the tooth mobility scale as a quantitative variable instead of a categorical variable, the mean evolution between baseline and 1 month after injection is - 0.78 (± 0.736).

| | Gel A n*=27 |
|---|---|
| 1 month after injection | |
| n | 23 |
| missing data | 4 |
| mean (standard deviation) | -0.78 (0.736) |
| median | -1.0 |
| min , max | -2 , 0 |

(continued)

| | Gel A n*=27 |
|---|---|
| 1 month after injection | |
| paired T-test P | <0.001 |
| *n= number of teeth | |

0= Ankylosis
1= Physiological mobility perceptible between two fingers
2= Transverse mobility visible to the naked eye
3= Transverse mobility > 1 mm
4= Axial mobility

There is thus a statistically significant improvement of the tooth mobility between baseline and visit at 1 month after injection for gel A according to the present invention.

Example 3 clearly shows that the gel A according to the invention is particularly appropriate for reducing the tooth mobility when injected in the periodontal pocket of a subject. Therefore, a gel according to the invention is particularly interesting for treating periodontal diseases.

## Claims

1. A sterile and injectable gel comprising at least one crosslinked hyaluronic acid or a salt thereof and at least one non-crosslinked hyaluronic acid or a salt thereof for use in preventing and/or treating periodontal diseases and/or associated troubles and disorders, wherein said gel has a cross-over stress greater than or equal to 400 Pa and/or a compressive strength greater than or equal to 20 N.s.

2. A sterile and injectable gel comprising at least one crosslinked hyaluronic acid or a salt thereof and at least one non-crosslinked hyaluronic acid or a salt thereof for use in filling void in a periodontal pocket, wherein said gel has a cross-over stress greater than or equal to 400 Pa and/or a compressive strength greater than or equal to 20 N.s.

3. A sterile and injectable gel comprising at least one crosslinked hyaluronic acid or a salt thereof and at least one non-crosslinked hyaluronic acid or a salt thereof for use as matrix material in a periodontal pocket, wherein said gel has a cross-over stress greater than or equal to 400 Pa and/or a compressive strength greater than or equal to 20 N.s.

4. A sterile and injectable gel comprising at least one crosslinked hyaluronic acid or a salt thereof and at least one non-crosslinked hyaluronic acid or a salt thereof for use in reducing the mobility of at least one tooth to the periodontal pocket and/or improving the adhesion of the gum with at least one tooth, wherein said gel has a cross-over stress greater than or equal to 400 Pa and/or a compressive strength greater than or equal to 20 N.s.

5. The gel according to anyone of claims 1 to 4 wherein said gel also has an extrusion force lower than or equal to 25 N, in particular via a 27G needle.

6. The gel according to any one of claims 1 to 5, wherein it is obtained by a method comprising at least the steps of:

   a) contacting, at least one crosslinked hyaluronic acid or a salt thereof with at least one non-crosslinked hyaluronic acid or a salt thereof, said crosslinked and non-crosslinked hyaluronic acid being, independently one from the other, in a dry state or in a hydrated state, and
   b) mixing them until homogenization, and where applicable adding an aqueous solution, for forming a hydrogel having a cross-over stress greater than or equal to 400 Pa and/or a compressive strength greater than or equal to 20 N.s, and preferably an extrusion force lower than or equal to 25 N, in particular via a 27G needle.

7. The gel according to the previous claim wherein the non-crosslinked acid and the crosslinked hyaluronic acid contacted in step a) are both in a hydrated state, in particular they are under the form of homogenous hydrogels that have preferably the same concentration in hyaluronic acid.

8. The gel according to the previous claim wherein the homogenous hydrogel of crosslinked hyaluronic acid or a salt thereof has a cross-over stress greater than or equal to 400 Pa and/or a compressive strength greater than or equal to 20 N.s and an extrusion force greater than 25 N, in particular via a 27G needle.

9. The gel according to claim 6 wherein in step a) the non-crosslinked hyaluronic acid is provided diluted in an aqueous solution and the crosslinked hyaluronic acid is provided as a solid and dense three-dimensional network as obtained just after the crosslinking of the non-crosslinked hyaluronic acid raw material and before swelling to the desired concentration of hyaluronic acid.

10. The gel according to the any one of claims 1 to 9 wherein the weight ratio of the non-crosslinked hyaluronic acid over the crosslinked hyaluronic acid is ranging from 0.001 to 0.25, preferably from 0.01 to 0.11, more preferably from 0.03 to 0.05.

11. The gel according to any one of claims 1 to 10, wherein the crosslinked hyaluronic acid or salt thereof is obtained by crosslinking at least one non-crosslinked hyaluronic acid or a salt thereof with at least one crosslinking agent, said non-crosslinked hyaluronic acid having a molecular weight ranging preferably from 0.3 MDa to 10 MDa, more preferably ranging from 0.5 MDa to 4 MDa, more preferably ranging from 0.5 to 2 MDa.

12. The gel according to claim 11, wherein the crosslinking agent is selected from the group consisting of 1,4-butanediol diglycidyl ether (BDDE), diepoxyoctane or 1,2-bis(2,3-epoxypropyl)-2,3-ethylene, 1,4-bis(2,3-epoxypropoxy)butane, 1,4-bisglycidyloxybutane, 1,2-bis(2,3-epoxypropoxy)ethylene, and 1-(2,3-epoxypropyl)-2,3-epoxycyclohexane, endogenous polyamines, and mixtures thereof and is preferably 1,4-butanediol diglycidyl ether.

13. The gel according to any one of claims 1 to 12, wherein the crosslinked hyaluronic acid or salt thereof has a degree of crosslinking ranging from 0.1% to 20%, preferably from 3% to 12%, more preferably from 9 to 11%.

14. The gel according to any one of claims 1 to 13, wherein the amount of crosslinked hyaluronic acid or salt thereof is ranging from 80% to 99.9% by weight, preferably from 90% to 99% by weight, more preferably from 95% to 97% by weight, relative to the total weight of hyaluronic acid in the gel.

15. The gel according to any one of claims 1 to 14, wherein the amount of non-crosslinked hyaluronic acid or salt thereof is ranging from 0.1% to 20% by weight, preferably from 1% to 10%, more preferably from 3% to 5% by weight, relative to the total weight of hyaluronic acid in the gel.

16. The gel according to any one of claims 1 to 15, wherein the gel comprises from 2% to 3% by weight of hyaluronic acid, relative to the total weight of the gel, consisting in:

- from 90% to 97% by weight of crosslinked hyaluronic acid or a salt thereof having a degree of crosslinking ranging from 9% to 11%, relative to the total weight of hyaluronic acid, and
- from 3% to 10% by weight of non-crosslinked hyaluronic acid or a salt thereof, relative to the total weight of hyaluronic acid.

17. The gel according to any one of claims 1 to 16, wherein the gel further comprises at least one compound, particularly an active material, such as a therapeutic agent like an anesthetic agent, an antibiotic agent, an antioxidant, a vitamin and mixture thereof.

18. A use of a sterile and injectable hydrogel comprising at least one crosslinked hyaluronic acid or a salt thereof and at least one non-crosslinked hyaluronic acid or a salt thereof for preventing and/or treating periodontal diseases and/or associated troubles and disorders, wherein said gel has a cross-over stress greater than or equal to 400 Pa and/or a compressive strength greater than or equal to 20 N.s, and preferably an extrusion force lower than or equal to 25 N, in particular via a 27G needle.

19. A use of a sterile and injectable gel comprising at least one crosslinked hyaluronic acid or a salt thereof and at least one non-crosslinked hyaluronic acid or a salt thereof for filling, in particular as a matrix material, a periodontal pocket, wherein said gel has a cross-over stress greater than or equal to 400 Pa and/or a compressive strength greater than or equal to 20 N.s, and preferably an extrusion force lower than or equal to 25 N, in particular via a 27G needle.

20. A use of a sterile and injectable gel comprising at least one crosslinked hyaluronic acid or a salt thereof and at least

one non-crosslinked hyaluronic acid or a salt thereof for reducing the mobility of at least one tooth to the periodontal pocket and/or improving the adhesion of the gum with at least one tooth wherein said gel has a cross-over stress greater than or equal to 400 Pa and/or a compressive strength greater than or equal to 20 N.s, and preferably an extrusion force lower than or equal to 25 N, in particular via a 27G needle.

**21.** A use of a sterile and injectable gel comprising at least one crosslinked hyaluronic acid or a salt thereof and at least one non-crosslinked hyaluronic acid or a salt thereof for forming a medium appropriate to the regeneration of cells in a periodontal pocket wherein said gel has a cross-over stress greater than or equal to 400 Pa, and/or a compressive strength greater than or equal to 20 N.s, and preferably an extrusion force lower than or equal to 25 N, in particular via a 27G needle.

**22.** The use according to claim 17 to 21, wherein said gel is as defined in any one of claims 5 to 17.

**23.** A process for improving the injectability of a gel comprising at least one crosslinked hyaluronic acid or a salt thereof and having a cross-over stress greater than or equal to 400 Pa and/or a compressive strength greater than or equal to 20 N.s and which value of cross-over stress and/or compressive strength have to be maintained greater than or equal respectively to 400 Pa and 20 N.s., comprising the step of adding to said gel an efficient amount of at least one gel of hyaluronic acid whose hyaluronic acid component consists in non-crosslinked hyaluronic acid or a salt thereof.

**24.** The process of the previous claim wherein the injectability is adjusted to an extrusion force lower than or equal to 25 N, in particular via a 27G needle.

Europäisches Patentamt
European Patent Office
Office européen des brevets

# EUROPEAN SEARCH REPORT

Application Number
EP 17 30 5105

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2015/038457 A1 (BOURDON FRANCOIS [FR] ET AL) 5 February 2015 (2015-02-05)<br>* claims; examples *<br>* paragraphs [0022] - [0025], [0034] - [0087], [0072] - [0079], [0171], [0176] - [0190], [0200] - [0210], [0219], [0224] *<br>----- | 1-24 | INV.<br>A61K9/00<br>A61K47/36<br>A61K38/10<br>A61L27/20 |
| X | US 2014/088037 A1 (BON BETEMPS JEREMIE [FR] ET AL) 27 March 2014 (2014-03-27)<br>* claims; examples *<br>* paragraphs [0065] - [0068], [0104] - [0150], [0165] *<br>----- | 1-24 | |
| X | US 2016/144043 A1 (TAUZIN BÉNÉDICTE VINCENTE [FR]) 26 May 2016 (2016-05-26)<br>* paragraphs [0054], [0059], [0061], [0104] - [107137] *<br>* claims; examples *<br>----- | 1-24 | |
| X | WO 2016/096920 A1 (TEOXANE [CH]) 23 June 2016 (2016-06-23)<br>* the whole document *<br>----- | 1-6,11, 12,18-24 | TECHNICAL FIELDS SEARCHED (IPC)<br>A61K<br>A61L |
| A | EP 0 914 832 A1 (KURARAY CO [JP]) 12 May 1999 (1999-05-12)<br>* the whole document *<br>----- | 1-24 | |
| A | US 2015/232623 A1 (BARG HEIKO [DE] ET AL) 20 August 2015 (2015-08-20)<br>* the whole document *<br>----- | 1-24 | |

The present search report has been drawn up for all claims

1

| Place of search | Date of completion of the search | Examiner |
|---|---|---|
| The Hague | 18 July 2017 | Ceyte, Mathilde |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 17 30 5105

This annex lists the patent family members relating to the patent documents cited in the above-mentioned European search report. The members are as contained in the European Patent Office EDP file on
The European Patent Office is in no way liable for these particulars which are merely given for the purpose of information.

18-07-2017

| Patent document cited in search report | Publication date | Patent family member(s) | Publication date |
|---|---|---|---|
| US 2015038457 A1 | 05-02-2015 | AU 2014298046 A1 | 10-03-2016 |
| | | CA 2919792 A1 | 05-02-2015 |
| | | CN 105682657 A | 15-06-2016 |
| | | EP 3027186 A1 | 08-06-2016 |
| | | JP 2016527264 A | 08-09-2016 |
| | | KR 20160038047 A | 06-04-2016 |
| | | US 2015038457 A1 | 05-02-2015 |
| | | US 2016166554 A1 | 16-06-2016 |
| | | US 2016325021 A1 | 10-11-2016 |
| | | WO 2015015407 A1 | 05-02-2015 |
| US 2014088037 A1 | 27-03-2014 | AU 2013307648 A1 | 12-03-2015 |
| | | CA 2881750 A1 | 06-03-2014 |
| | | CN 104582672 A | 29-04-2015 |
| | | CN 106890360 A | 27-06-2017 |
| | | CR 20150174 A | 27-08-2015 |
| | | EP 2890360 A1 | 08-07-2015 |
| | | FR 2994846 A1 | 07-03-2014 |
| | | HK 1208350 A1 | 04-03-2016 |
| | | JP 2015526489 A | 10-09-2015 |
| | | KR 20150046307 A | 29-04-2015 |
| | | RU 2015111247 A | 27-10-2016 |
| | | SG 11201501215Y A | 28-05-2015 |
| | | US 2014088037 A1 | 27-03-2014 |
| | | US 2016303024 A1 | 20-10-2016 |
| | | WO 2014032804 A1 | 06-03-2014 |
| US 2016144043 A1 | 26-05-2016 | AU 2014280544 A1 | 03-12-2015 |
| | | CA 2914417 A1 | 18-12-2014 |
| | | CN 105358186 A | 24-02-2016 |
| | | EP 3007737 A1 | 20-04-2016 |
| | | FR 3006689 A1 | 12-12-2014 |
| | | HK 1221669 A1 | 09-06-2017 |
| | | JP 2016527338 A | 08-09-2016 |
| | | KR 20160031465 A | 22-03-2016 |
| | | SG 11201510103Y A | 28-01-2016 |
| | | US 2016144043 A1 | 26-05-2016 |
| | | WO 2014199022 A1 | 18-12-2014 |
| WO 2016096920 A1 | 23-06-2016 | FR 3029928 A1 | 17-06-2016 |
| | | WO 2016096920 A1 | 23-06-2016 |
| EP 0914832 A1 | 12-05-1999 | DE 69836461 T2 | 13-09-2007 |
| | | EP 0914832 A1 | 12-05-1999 |
| | | JP 4177900 B2 | 05-11-2008 |
| | | WO 9844950 A1 | 15-10-1998 |

EPO FORM P0459

For more details about this annex : see Official Journal of the European Patent Office, No. 12/82

page 1 of 2

## ANNEX TO THE EUROPEAN SEARCH REPORT ON EUROPEAN PATENT APPLICATION NO.

EP 17 30 5105

This annex lists the patent family members relating to the patent documents cited in the above-mentioned European search report. The members are as contained in the European Patent Office EDP file on
The European Patent Office is in no way liable for these particulars which are merely given for the purpose of information.

18-07-2017

| Patent document cited in search report | Publication date | Patent family member(s) | Publication date |
| --- | --- | --- | --- |
| US 2015232623 A1 | 20-08-2015 | AR 091454 A1 | 04-02-2015 |
| | | AU 2013276844 A1 | 18-12-2014 |
| | | CA 2876070 A1 | 19-12-2013 |
| | | CN 104395348 A | 04-03-2015 |
| | | EP 2861626 A1 | 22-04-2015 |
| | | HK 1205750 A1 | 24-12-2015 |
| | | JP 2015526537 A | 10-09-2015 |
| | | KR 20150022934 A | 04-03-2015 |
| | | RU 2015101105 A | 10-08-2016 |
| | | SG 11201408312U A | 29-01-2015 |
| | | TW 201404409 A | 01-02-2014 |
| | | US 2015232623 A1 | 20-08-2015 |
| | | WO 2013185934 A1 | 19-12-2013 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2009144708 A
- WO 2014056723 A
- US 9353194 B


### Non-patent literature cited in the description


- Anderson's Pathology. 1992, 2000
- Anderson's Pathology. 1992, 1999-2000
- **SHAFER et al.** *A Textbook of Oral Pathology,* 1983
- **TAMMI R. ; AGREN UM. ; TUHKANEN AL. ; TAMMI M.** Hyaluronan metabolism in skin. *Progress in Histochemistry & Cytochemistry,* 1994, vol. 29 (2), 1-81